Europäisches Patentamt

European Patent Office

Office européen des brevets

(19)

(11) Publication number: **0 227 278**
**A2**

(12) # EUROPEAN PATENT APPLICATION

(21) Application number: 86308717.7

(51) Int. Cl.⁴ **A61M 5/00**

(22) Date of filing: 07.11.86

(30) Priority: 18.11.85 US 798936

(43) Date of publication of application:
**01.07.87 Bulletin 87/27**

(84) Designated Contracting States:
**AT BE CH DE FR GB IT LI LU NL SE**

(71) Applicant: **Carbomedics Inc.**
**1300 East Anderson Lane**
**Austin Texas 78752(US)**

(72) Inventor: **Ellis, Willard Harrison**
**2804 Live Oak**
**Round Rock Texas 78681(US)**

(74) Representative: **Lucas, Brian Ronald**
**Lucas, George & Co. 135 Westhall Road**
**Warlingham Surrey CR3 9HJ(GB)**

(54) **A cap for use with a percutaneous device.**

(57) A cap (18,18a,18b ) for use with a percutaneous device (10,10a,10b ). The cap (18,18a,18b) has a surface (21, 21a,21b) carrying an adhesive for cutaneous contact in juxtaposition with the percutaneous device - (10,10a,10b) so that relative movement between the percutraneous device (10,10a,10b) and the skin - (12,12a,12b) is inhibited.

FIG. 3

EP 0 227 278 A2

## A Cap for use with a Percutaneous Device

This invention relates to a cap for use with a percutaneous device.

Various percutaneous devices are known for long-term or permanent access through the skin. A typical percutaneous device is a blood-access device that may be opened and closed repetitively for dialysis purposes. Other percutaneous medical devices include infusion pumps, conduits for long-term delivery of drugs, devices for nerve stimulation, etc. In the use of these devices, it is important that movement of the skin relative to such devices be eliminated or inhibited so as to reduce cutaneous trauma. Indeed, in many instances, it is important that the skin immediately adjacent the percutaneous device heal and remain healthy to minimize the possibility of bacterial infection. At the same time, it is important that the skin around these devices have access to air.

Unfortunately, permanently implanted percutaneous devices commonly develop exit site infections which often necessitates removal of a functioning device. Relative movement between the device-tissue interface inhibits tissue attachment to the device, and the resulting poor seal allows infectious organisms to penetrate into the seal interface.

Various approaches have been proposed to prevent the trauma noted above such as surgically reinforcing the skin tissue around the opening so that the stress is concentrated elsewhere and the interface between the percutaneous device and the skin is protected. However, the surgical procedures involved can be tedious and are without assurances of success.

According to the present invention there is provided for use with a percutaneous device having a penetrating portion which, in use, extends through the skin, a cap having a surface for cutaneous contact circumjacent the penetrating portion, and adhesive means for removably affixing said cap to skin circumjacent said penetrating portion so that, in use, relative movement between said percutaneous device and the skin circumjacent thereto is inhibited.

Preferably, said surface is annular.

In one embodiment the cap includes means to snugly accommodate, in use, the free extremity of said penetrating portion.

Preferably, the cap includes a section removeable, in use, to gain access to said penetrating portion.

Advantageously, the section includes a recess which snugly accommodates, in use, the free extremity of said penetrating portion.

In another embodiment, the cap includes an opening to engage, in use, the side(s) of a penetrating portion projecting therethrough.

Preferably, at least a part of caps in accordance with the invention is transparent or sufficiently transparent to enable, in use, the condition of the skin surrounding said penetrating portion to be observed without removal of said cap.

Advantageously, caps in acoordance with the invention include one or more filters which, in use, permit passage of air through said cap to the skin surrounding said penetrating portion but inhibit the penetration of infectious organisms.

Caps in accordance with the invention may also be provided with a mounting band to further facilitate attachment to the body.

The cap may comprise a band which has the surface for cutaneous contact circumjacent the penetrating portion, and a cover secured to said band.

There is also provided, in combination, a cap in accordance with the invention and a percutaneous device.

The present invention also provides a method for inhibiting relative movement between a percutaneous device having a penetrating portion extending through the skin, and the skin circumjacent thereto, which method comprises the steps of:

(a) removably adhering a first cap according to the invention to the skin circumjacent the penetrating portion;

(b) subsequently removing the first cap; and

(c) removably adhering a second cap according to the invention to the skin circumjacent the penetrating portion;

wherein the first and second caps are shaped and dimensioned so that at least a mojor portion of the skin initially in contact with the first cap is not in contact with the second cap.

This procedure is simple, relatively inexpensive and dispenses with the more cumbersome surgical reinforcement of tissue around the opening for the percutaneous device.

For a better understanding of the invention reference will now be made, by way of example, to the accompanying drawings, in which:-

Figure I is an elevation view, partly in cross section, of one embodiment of a cap in accordance with the invention and a percutaneous device in use;

Figure 2 is an elevation view, partly in cross section, illustrating a second embodiment of a cap in accordance with the invention and a percutaneous device in use;

Figure 3 is an elevation view, partly in cross section, illustrating a third embodiment of a cap in accordance with the invention and a percutaneous device in use, wherein the cap is provided with filters for the passage of air therethrough; and

Figure 4 is a plan view of the arrangement shown in Figure 3 provided with a mounting strap.

Infections commonly develop where the penetrating portion of percutaneous devices pass through the skin. This is largely due to relative relative movement between the penetrating portion and the surrounding skin which inhibits satisfactory tissue attachment.

A percutaneous device 10 in the form of a blood access device is illustrated in Figure I.

The percutaneous device 10 is implanted subcutaneously, the reference character 12 indicating skin or cutaneous layers and the reference character 14 indicating subcutaneous layers of body tissue. It is important that the skin 12 immediately adjacent the penetrating portion 16 of the percutaneous device 10 be attached firmly to the penetrating portion 16 and, once attached, remain so. Any relative movement between the skin 12 and the percutaneous device 10 naturally tends to dislodge the skin from about the penetrating portion 16 of the percutaneous device resulting in a poor seal.

Caps in accordance with the present invention inhibit the relative movement described above. In particular cap 18 comprises a cover 22 and band 20 of annular shape secured thereto. The band 20 has a surface 21 which is secured to the skin 12 circumjacent the penetrating portion 16 of the percutaneous device 10 to a layer of adhesive 24. Any suitable adhesive may be used so long as it permits the cap 18 to be firmly affixed to the skin 12 and yet is removable without undue irritation to the skin 12 and without unduly jeopardizing tissue attachment to the penetrating portion 26 of the percutaneous device 10. An example of a suitable adhesive that may be employed for this purpose is sold under the trademark STOMAHESIVE by Convatech, P.O. Box 4000, Princeton, New Jersey 08540.

While the band 20 is annular and cover 22 dome shaped in the embodiment illustrated in Figure I, it will be understood that the band 20 may have any other suitable curvilinear or even a multisided geometric configuration. Also, the cover 22 may have a configuration other than the dome shape illustrated in Figure I. Similarly, the dimensions of the band 20 and cover 22 may vary but should remain within limits such that, upon adhesion of the cap 18 to the skin 12, relative movement between the penetrating portion 16 of the percutaneous device 10 and the skin circumjacent thereto is inhibited.

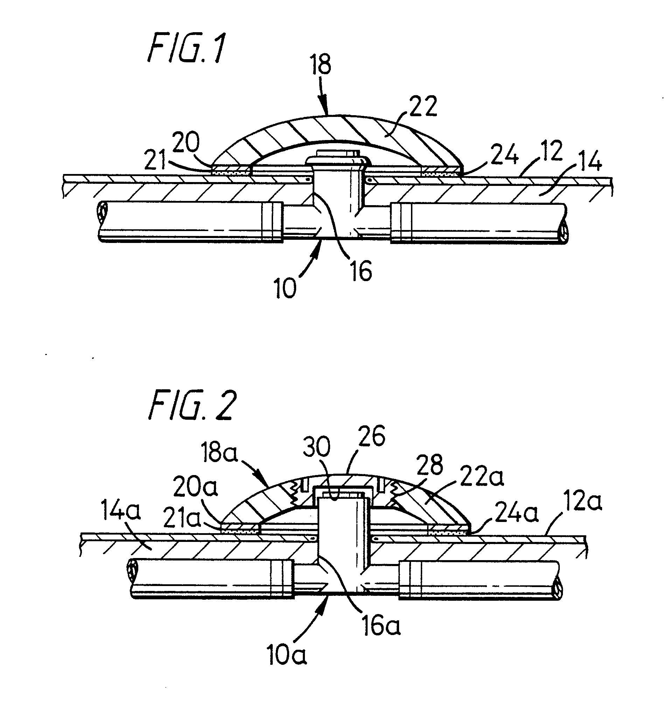

The cap 18 $\underline{a}$ illustrated in Figure 2 is similar to the cap 18 showsn in Figure I. However, the cover 22a is provided with a center access section 26 having threads 28 that threadably engage similar threads formed in the cover 22$\underline{a}$. Thus, the access section 26 may be opened so as to carry out the necessary functions vis-a-vis the percutaneous device 10$\underline{a}$ without removing the remaining portion of the cap 18$\underline{a}$. This feature further reduces the risk of damage to the interface of the skin 12 and the penetrating portion 16$\underline{a}$ of the percutaneous device 10$\underline{a}$.

Again with respect to the embodiment of Figure 2, the centre access section 26 of the cover 22$\underline{a}$ is provided with a recess 30 which snugly accommodates the extremity of the penetrating portion 16$\underline{a}$ of the percutaneous device 10$\underline{a}$. The recess 30 is only slightly larger than the outer extremity of the penetrating portion 16$\underline{a}$ of the percutaneous device 10$\underline{a}$. As a result, any significant movement of the cap 18$\underline{a}$, the skin 12$\underline{a}$ to which it is attached, and the percutaneous device 10$\underline{a}$ occurs in unison to promote an even more stable interface between the skin 12$\underline{a}$ and the percutaneous device 10$\underline{a}$. It will be appreciated that the recess 30 may be provided in the embodiment of Figure I and is not dependent on the use or not of the access section 26.

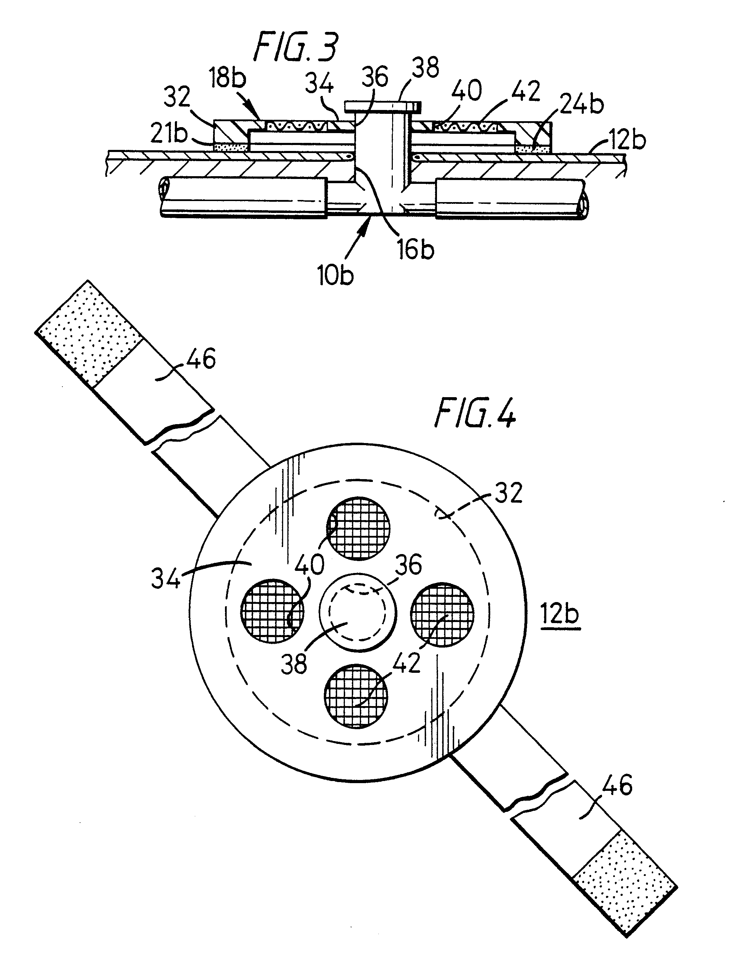

Referring now to the embodiment shown in Figures 3 and 4, the cover 34 is generally flat and is provided with a central opening 36 to receive the penetrating portion 16$\underline{b}$ of the percutaneous device 10$\underline{b}$. In this embodiment, the penetrating portion 16$\underline{b}$ of the percutaneous device 10$\underline{b}$ has a cap member 38. In this configuration, the opening 36 of the cover 34 engages the penetrating portion 16$\underline{b}$ of the percutaneous device 10$\underline{b}$ which ensures that the skin 12 $\underline{b}$ and the cap 18$\underline{b}$ move as a unit with the percutaneous device 10$\underline{b}$.

The embodiment illustrated in Figures 3 and 4 provides the additional feature of permitting the passage of air through the cap 18$\underline{b}$ but restricting penetration of infectious organisms. This is accomplished through the provision of a plurality of holes 40 formed in the cover 34, the holes 40 being sealed with filters made from a suitable cloth or paper-like material 42 that permits movement of air therethrough but inhibits penetration of infectious organisms and other foreign bodies. Examples of suitable filter materials that may be employed for this purpose are sold under the trademarks HYDROPHOB and DURAPORE by Millipore, South San Francisco, California. Thus, the portion of the skin 12$\underline{b}$ circumjacent the penetrating portion 16 $\underline{b}$ and beneath the cap 18$\underline{b}$ has access to air so as to permit a healthy skin condition and tissue interface with the penetrating portion 16$\underline{b}$ of the percutaneous device 10$\underline{b}$.

Figure 4 illustrates the optional feature of a mounting strap 46 suitably secured to the cap 18b. The mounting strap 46 may extend about a body extremity such as an arm to further secure the cap 18b in position. VELCRO (Registered Trade Mark) may be used for purposes of joining the ends of the strap 46.

The caps described are disposable items in the sense that they are intended to be discarded after a period of use and replaced with a similar device. A distinct advantage is that, upon replacement of a cap, the replacement unit may have a skin engaging portion of a diameter different from the diameter of the preceding cap for adhesion to a substantially different skin area. This allows the skin previously in contact with the adhesive of the cap to regenerate to a healthy state, and successive use of caps of differing diameters promotes overall good health of the skin area around the percutaneous device.VThe band and cover of the stabilizer cap may be formed either separately (as shown in Figures 1 and 2) or integrally (as shown in Figures 3 and 4) of materials such as POLYSULFONE - (trademark for a resin manufactured and marketed by Union Carbide Corporation), polycarbonate and other such durable materials. The width of the band for purposes of receiving the adhesive material will vary depending upon circumstances of use. A width of 3 mm has been found acceptable. The overall diameter of the cap (determined by the outside diameter of the band) likewise may vary, but approximately 35 mm has been found suitable. Variations of such diameter may be made to promote skin health as previously explained. Finally, the overall height of the cap (i.e. the distance from the highest point of the outside surface of the cover to the lower surface of the band which carries the adhesive material) may vary, approximately 10 mm being suitable. Of course, the height dimension will vary amoung embodiments illustrated in the drawings.

It may be desirable in some instances to form the cap, or at least the cover portion thereof, of a transparent or sufficiently translucent material as to permit viewing of skin conditions thereunder. This will permit frequent inspection of not only the skin condition but also the interface of skin tissue with the percutaneous device.

## Claims

1. For use with a percutaneous device - (10,10a,10b) having a penetrating portion (16,16a,16b) which, in use, extends through the skin (12,12a,12b) a cap (18,18a,18b having a surface (21,21a2lb) for cutaneous contact circumjacent the penetrating portion, and adhesive means (24,24a,24b) for re-

movably affixing said cap (18,18a,18b) to skin - (12,12a,12 b) circumjacent said penetrating portion so that, in use, relative movement between said percutaneous device (10,10a,10 b) and the skin - (12,12a,12b) circumjacent thereto is inhibited.

2. A cap as claimed in Claim 1, wherein said surface is annular.

3. A cap as claimed in Claim 1 or 2, including means (30) to snugly accommodate, in use, the free extremity of said penetrating portion (16a).

4. A cap as claimed in Claim 1, 2 or 3, including a section (26) removable, in use, to gain access to said penetrating portion (16a.

5. A cap as claimed in Claim 4 when appended to Claim 3, wherein said section (26) includes a recess (30) which snugly accommodates, in use, the free extremity of said penetrating portion (16a).

6. A cap as claimed in Claim 1 or 2, including an opening (36) to engage, in use, the side(s) of a penetrating portion (16b) projecting therethrough.

7. A cap as claimed in any preceding Claim, at least part of which is transparent or sufficiently transparent to enable, in use, the condition of the skin surrounding said penetrating portion to be observed without removal of said cap.

8. A cap as claimed in any preceding Claim, including one or more filters (42) which, in use, permit passage of air through said cap (18b) to the skin surrounding said penetrating portion (16b) but inhibit the penetration of infectious organisms.

9. A cap as claimed in any preceding claim comprising a band (20,20a) which has the surface - (21,21a , for cutaneous contact circumjacent the penetrating portion (16,16 a), and a cover (22,22a) secured to said band.

10. A cap as claimed in any preceding Claim, including a mounting band (46).

11. In combination, a cap as calimed in any preceding Claim and a percutaneous device.

12. A method for inhibiting relative movement between a percutaneous device having a penetrating portion extending through the skin and the skin circumjacent thereto, which method comprises the steps of:

(a) removable adhering a first cap - (18,18a,18b) according to any one of Claims 1 to 9 to the skin circumjacent the penetrating portion;

(b) subsequently removing said first cap - (18,18a,18b ); and

(c) removably adhering a second cap - (18,18a,18b) according to any one of Claims 1 to 9 to the skin circumjacent the penetrating portion; wherein said first and second caps are shaped and dimensioned so that at least a major portion of the skin initially in contact with said first cap is not in contact with said second cap.

*FIG.1*

*FIG.2*

FIG.3

FIG.4